Europäisches Patentamt

European Patent Office

Office européen des brevets

(19)

(11) **EP 1 209 610 A1**

(12) **DEMANDE DE BREVET EUROPEEN**

(43) Date de publication:
**29.05.2002 Bulletin 2002/22**

(51) Int Cl.7: **G06F 19/00**

(21) Numéro de dépôt: **00403326.2**

(22) Date de dépôt: **28.11.2000**

(84) Etats contractants désignés:
**AT BE CH CY DE DK ES FI FR GB GR IE IT LI LU
MC NL PT SE TR**
Etats d'extension désignés:
**AL LT LV MK RO SI**

(71) Demandeur: **Valentin Capital Management
2128 Luxembourg Ville (LU)**

(72) Inventeurs:
• **Sayada, Chalom
75020 Paris (FR)**
• **Halfon, Philippe
13008 Marseille (FR)**

(74) Mandataire: **Vaillant, Jeanne et al
Ernest Gutmann - Yves Plasseraud SA,
3, rue Chauveau-Lagarde
75008 Paris (FR)**

(54) **Dispositif et procédé de détermination de données d'affinité entre une cible et un ligand**

(57)     Un dispositif de détermination de donnée d'affinité entre un ligand et une cible comprend des moyens de calcul (1) pour recevoir des informations représentatives d'une cible et d'un ligand, susceptible d'interagir avec la cible en l'un de ses sites récepteurs, et déterminer une valeur d'affinité représentative de l'énergie d'association entre cette cible et ce ligand à partir des informations reçues, et des moyens de comparaison (3) agencés pour délivrer une donnée représentative d'un niveau d'affinité entre la cible et le ligand à partir de la valeur d'affinité cible/ligand et d'un paramètre de référence choisi.

**FIG. 2**

EP 1 209 610 A1

**Description**

L'invention concerne le domaine de la modélisation d'interactions entre des cibles et des ligands.

On entend ici par « cible », tout agent pathogène d'un organisme humain, animal ou végétal; il peut s'agir aussi bien de (macro)molécules, de microorganismes pathogènes tels que les virus, les bactéries, les parasites ou les champignons, que de cellules infectées par un virus ou un parasite.

On entend par « ligand » tout type de structure susceptible de s'associer spécifiquement à un site récepteur d'une cible pour en bloquer ou en réduire l'activité, ou bien pour en modifier les propriétés. Il pourra donc s'agir d'une molécule chimique ou d'une macro-molécule chimique ou biologique ou d'une association de ceux-ci, ou plus généralement de toute substance à visée thérapeutique ou prophylactique, telle qu'un médicament de thérapie génique ou de thérapie cellulaire.

On entend par « site » un ou plusieurs endroits d'une cible, qui définissent une zone ou bien une région avec laquelle un ligand peut interagir. L'interaction comprend tout type d'association ou de liaison susceptible de modifier les propriétés ou paramètres biologiques ou physico-chimiques de la cible.

Enfin, on entend ici par « modélisation » la détermination par le calcul de certains paramètres ou certaines propriétés biologiques ou physico-chimiques de cibles ou d'entités formées par l'association d'une cible et d'un ligand.

Dans de nombreux domaines, il est important de pouvoir prédire si un ligand est susceptible d'interagir avec une cible. Cela est encore plus important dans le domaine médical, notamment lorsqu'une maladie évolue rapidement. Actuellement, il n'est pas possible de quantifier l'association entre une cible et un ligand autrement que par comparaison avec d'autres associations. Ainsi, dans le cas d'un virus (cible) on peut dire qu'il présente une résistance vis-à-vis d'un premier médicament (ligand) qui est X fois supérieure à celle qu'il présente vis-à-vis d'un second médicament. En d'autres termes, la durée d'association de la cible avec le premier ligand est X fois plus courte que celle de cette cible avec le second ligand.

L'homme de l'art détermine la résistance d'une entité cible/ligand, ou plus généralement de tout autre paramètre d'affinité représentatif d'une association cible/ligand, par des tests *in vivo* ou *in vitro*. Or, que le test soit effectué *in vivo* ou *in vitro,* il est fréquent que l'environnement particulier dans lequel il s'effectue influence les résultats. Les résistances ainsi obtenues peuvent donc varier sensiblement d'un test à l'autre, et d'un sujet à l'autre. Pour cette raison, notamment, on ne peut généralement trouver dans la littérature spécialisée que les valeurs moyennes statistiques des résistances, ou de tout autre paramètre d'affinité représentatif d'une association cible/ligand. Par ailleurs, certains tests *in vitro* d'identification d'interactions cible/ligand nécessitent la mise en oeuvre de techniques complexes, telles que la culture cellulaire, le séquençage ou le phénotypage, qui sont onéreux et qui peuvent nécessiter plusieurs semaines de travail, ce qui peut être incompatible avec la fréquence de mutation d'un virus (cible), comme dans le cas du virus HIV-1 (virus d'immuno-déficience humaine de type 1), et plus précisément de la protéase et de la transcriptase inverse (TI) du VIH-1. Ces cibles peuvent être inhibées (ou neutralisées) par des inhibiteurs (ligands).

Dans le cas de la protéase du VIH-1, on connaît des médicaments inhibiteurs qui bloquent la phase tardive de la maturation virale, et plus précisément agissent sur la réplication à l'intérieur des cellules chroniquement infectées. Ils sont actifs sur les lymphocytes CD4 activés qui produisent du virus, mais également sur des cellules simplement présentatrices d'antigènes tels que les macrophages. Ainsi, la protéase du VIH-1 clive les polypeptides précurseurs produits par les gènes « gag » et « pol », lesquels permettent de générer les protéines structurales et enzymatiques du virion. En présence d'un inhibiteur de protéase du VIH-1, les virions qui sont produits sont immatures et donc incapables d'infecter de nouvelles cellules.

Parmi les inhibiteurs de la protéase du VIH, on pourra citer, notamment, l'indinavir, le ritonavir, le saquinavir, le nelfinavir, l'amprenavir, etc. Tous ces inhibiteurs de protéase sont des médicaments présentant un métabolisme prenant la voie des cytochromes p450. C'est pourquoi ils sont sensibles aux interactions médicamenteuses avec d'autres produits utilisant les mêmes voies métaboliques, ou induisent de telles interactions médicamenteuses.

En fait, d'une façon générale, un ligand (tel qu'un inhibiteur) interagit avec une cible (telle que la protéase ou la transcriptase inverse du VIH-1), au niveau de l'un au moins des sites récepteurs de cette dernière. Un tel site récepteur peut être défini par une partie continue de la cible, ou par plusieurs parties discontinues de cette cible, par exemple lorsque ces parties sont voisines du fait d'un repliement. Dans le cas de la protéase du VIH-1, un site récepteur est défini par un certain nombre d'acides aminés, appelés traditionnellement résidus. Ce sont ces résidus qui sont impliqués directement ou indirectement dans l'interaction (ou association) cible/ligand.

Comme indiqué précédemment, l'un des inconvénients majeurs que présente la protéase du VIH-1, c'est son aptitude à muter rapidement (en fait ce sont certains résidus qui subissent des mutations). Il en résulte qu'un médicament (tel qu'un inhibiteur) qui est réputé efficace vis-à-vis de la protéase de référence non mutée du VIH-1, est souvent partiellement inefficace, voire totalement inefficace, vis-à-vis de l'une au moins des mutations de cette protéase de référence ; en d'autres termes la cible (ici la protéase mutée) développe une résistance vis-à-vis du ligand (ici un médicament inhibiteur).

Il existe des listes dans lesquelles sont répertoriées les mutations connues de la protéase du VIH. Une telle liste contient par exemple les mutations référencées M46L, I54L, A71T, 154V, L90M, V82S, 184T, V82A, 150V, et V82F. A titre d'exemple, le résidu référencé « VAL82 » devient le résidu référencé « PHE82 » lorsque survient la mutation référencée « V82F ». Une cible peut comporter plusieurs mutations impliquant plusieurs résidus.

A partir de tests expérimentaux effectués *in vivo* ou *in vitro,* il est possible de créer des tables de correspondance entre des protéases du VIH-1 non mutée et mutées et des informations relatives à leur résistance vis à vis de certains inhibiteurs (ou médicaments).

Ces tables, qui sont bien connues de l'homme du métier, ne fournissent généralement des informations pour une ou plusieurs mutations déjà connues et identifiées. Elles sont stockées, parfois, sur des supports papier, mais, plus généralement, dans des mémoires, du type de celle accessible sur le site Internet de l'Université de STANFORD, de manière à pouvoir être utilisées par un logiciel informatique, comme par exemple le logiciel « GRANT » (pour Genotypic Resistance ANalysis Tool) de la société française INFOBIOGEN. Plus précisément, en fournissant à ce logiciel la séquence d'une mutation répertoriée dans l'une de ses tables, on obtient en sortie, lorsqu'il existe, son paramètre d'affinité (résistance) avec une substance connue.

Ces tables contiennent de plus des informations qui reposent sur des statistiques relativement faibles, ne sont pas renouvelées fréquemment, et qui de surcroît utilisent des critères non standardisés. Par ailleurs, ces tables ne sont généralement utilisables qu'une fois que l'on connaît le phénotype de la protéase du VIH-1 du patient, ce qui prend actuellement plusieurs semaines et permet à la protéase du VIH-1 du patient de subir une ou plusieurs mutations. Ces mutations étant difficilement prévisibles, les résultats du phénotypage deviennent alors inutilisables. De plus, les phénotypes sont basés sur des méthodes développées localement, fastidieuses et non standardisées, ce qui les rend difficilement reproductibles. Enfin, du fait des nombreuses opérations impliquées par le phénotypage, les taux d'échec sont assez élevés, typiquement de l'ordre de 30%.

Sur un autre plan, il n'existe pas de procédé permettant de déterminer par le calcul, et indépendamment de facteurs environnementaux liés aux tests expérimentaux, une donnée d'affinité permettant de « quantifier » la capacité d'un ligand (par exemple un médicament) à s'associer à une cible, dont la composition ou la structure a été récemment identifiée, en un site récepteur choisi de cette cible.

La constatation selon laquelle un effet pharmacologique ou thérapeutique résulte d'une interaction spécifique d'une cible, comportant un site récepteur, avec un effecteur (ligand) a conduit au développement de technologies permettant l'identification expérimentale des ligands efficaces sur des cibles données. On citera en particulier, l'association de la chimie combinatoire, consistant à générer toute une famille de molécules à partir d'un squelette de base, et du criblage à haut débit, qui consiste à mettre en présence des ligands éventuels, notamment obtenus en chimie combinatoire, avec sa ou ses cibles et de mesurer l'importance de la réaction résultant de l'association cible/ligand. Ces technologies sont lourdes à mettre en oeuvre, tant par la quantité de matériels chimiques et/ou biologiques à utiliser en parallèle que par la complexité des dispositifs aptes à réaliser et à interpréter les expérimentations.

L'invention a pour but d'apporter une solution aux inconvénients présentés ci-avant, et plus particulièrement de permettre d'estimer (ou prédire) la capacité d'une cible à interagir avec un ligand en au moins un site récepteur choisi, et ce avant une éventuelle phase expérimentale de validation. Entre autres avantages, l'invention peut permettre, d'une part, une économie substantielle de temps et du nombre de structures cibles à tester expérimentalement pour identifier un ligand candidat pour le développement d'un médicament, et d'autre part, à un praticien de disposer très rapidement d'informations susceptibles de l'aider à choisir un médicament adapté au virus infectant son patient.

Elle propose à cet effet un dispositif de détermination de données d'interaction entre un ligand et une cible, dans lequel on prévoit :

* des moyens de calcul capables, à réception d'informations représentatives d'une cible et d'un ligand, susceptible d'interagir avec la cible en l'un de ses sites récepteurs, de déterminer une valeur d'affinité (ou d'association), reproductible, représentative de l'énergie d'association entre cette cible et ce ligand, et
* des moyens de comparaison délivrant une donnée représentative d'un niveau d'affinité entre la cible et le ligand à partir de la valeur d'affinité cible/ligand, déterminée par les moyens de calcul, et d'un paramètre de référence choisi.

Les moyens de calcul sont capables, d'une part, de modéliser (ou générer ou encore construire) non seulement la cible et/ou le ligand désigné(s) par les informations reçues, mais également une entité constituée de l'association d'une cible et d'un ligand et, d'autre part, d'extraire de la modélisation de l'entité une valeur représentative de l'énergie d'association entre la cible et le ligand.

La modélisation résulte notamment de calculs d'énergie libre et d'énergie de liaison ou de non-liaison. On entend donc, ici, par « extraire » l'opération consistant à rechercher des résultats choisis parmi les nombreux calculs effectués lors de la modélisation. Par ailleurs, le mot « association » doit être compris dans une définition large. Il vise tout type d'interaction de proximité et ne requiert pas obligatoirement une ou plusieurs liaisons physiques, au niveau d'un site récepteur, entre la cible et le ligand.

D'autre part, on entend par « information représentative d'une cible ou d'un ligand », soit la constitution chimique ou biochimique de la cible ou du ligand, soit leur description structurale et énergétique, soit encore leur simple désignation (dans ce cas, on utilise une bibliothèque de cibles et de ligands dans laquelle on peut aller chercher le détail de la constitution de la cible ou du ligand).

Le paramètre de référence choisi pour effectuer la comparaison peut être le même pour une série d'entités cible/ligand d'un même type, ou bien il peut varier d'une entité à l'autre lorsque l'on dispose de résultats expérimentaux, par exemple pour des entités de référence du même type que, ou d'un type similaire à, l'entité concernée. Par ailleurs, le paramètre de référence peut être une valeur numérique ou un intervalle de valeurs numériques.

Le dispositif selon l'invention est donc destiné à déterminer une valeur d'affinité de préférence sous la forme d'une énergie d'association cible/ligand, à partir des paramètres physico-chimiques ou biologiques de l'entité cible/ligand, puis en déduire une donnée d'affinité qui caractérise l'aptitude du ligand à interagir avec la cible, par une comparaison à une référence.

Grâce à l'invention, il est donc possible de s'affranchir de nombreux tests *in vivo* et *in vitro* ainsi que de l'influence de l'environnement sur les valeurs des paramètres d'affinité, et par conséquent d'obtenir très rapidement et à moindre coût des informations d'interaction (ou d'association) fiables, non biaisées, reproductibles, standardisées, et selon des délais compatibles avec un suivi clinique des patients.

Selon les besoins, les moyens de comparaison peuvent délivrer une donnée d'interaction (ou d'association) sous la forme d'un message d'avertissement ou d'un écart ou rapport entre le paramètre de référence choisi et un paramètre d'affinité déduit, notamment, de la valeur d'affinité déterminée.

On entend ici par « avertissement » un message du type « mauvaise association » ou « forte résistance », qui correspond par exemple à un résultat de rapport de comparaison strictement supérieur à 1 (un), « très bonne association » ou « faible résistance », qui correspond par exemple à un résultat de rapport de comparaison strictement inférieur à 1 (un), et « association possible» ou « résistance possible », qui correspond par exemple à un résultat de rapport de comparaison de l'ordre de 1 (un).

On obtient par conséquent des données d'affinité qui permettent de savoir non seulement si un ligand est susceptible d'interagir (ou de s'associer) avec une cible, mais surtout une information permettant de quantifier la qualité de l'interaction (ou association). Cette information peut alors être utilisée pour fournir une classification de ligands par ordre de résistance croissante, par exemple.

Le dispositif pourra comporter d'autres caractéristiques prises séparément ou en combinaison, et notamment :

* une mémoire permettant de stocker une pluralité de multiplets (ou n-uplets) comportant chacun au moins une information représentative d'une cible, une information représentative d'un ligand et un paramètre de référence pour cette association cible/ligand, et dans ce cas les moyens de comparaison sont agencés de manière à extraire de la mémoire, à réception d'une valeur d'affinité cible/ligand issue des moyens de calcul, le paramètre de référence qui correspond à l'association cible/ligand en vue d'effectuer leur comparaison. Il est alors particulièrement avantageux que cette mémoire puisse stocker, sous la forme d'une première table, des premières cibles, dites de référence (par exemple du fait qu'elles n'ont pas subies de modification ; on les appelle « cibles sauvages » ou en anglais « wild type target »), en correspondance de listes de secondes cibles, qui résultent de modifications subies par les premières cibles de référence. Dans cette situation, il est possible de déterminer la donnée d'affinité entre une cible modifiée (seconde cible) et un ligand en utilisant le paramètre de référence soit de l'association seconde cible/ligand lorsqu'il est connu, soit de l'association ligand/cible de référence (ou première cible). Pour ce faire, les moyens de calcul sont agencés de manière à extraire de la mémoire la première cible qui est associée à la seconde cible qu'ils reçoivent, par exemple conjointement avec un ligand, puis à déterminer des première et seconde valeurs d'affinité représentatives respectivement des énergies d'association entre la première cible et le ligand et entre la seconde cible et le ligand, et enfin à effectuer une soustraction entre ces première et seconde valeurs d'affinité. Bien entendu, les moyens de comparaison doivent alors être agencés de manière à délivrer une donnée représentative d'un niveau d'affinité entre la seconde cible et le ligand à partir du résultat de la soustraction effectuée par les moyens de calcul et du paramètre de référence choisi, ce dernier étant préférentiellement celui qui correspond à l'association entre la première cible et le ligand ;

* des moyens de sélection capables de délivrer aux moyens de calcul les informations représentatives des cibles et des ligands. Il pourra s'agir de moyens d'interface homme/machine couplés à un module logiciel destiné à chercher dans la mémoire les informations représentatives d'une cible et/ou d'un ligand, fournies par un opérateur local ou distant. Par exemple, lorsque l'opérateur ne fournit que le nom d'une seconde cible, les moyens de sélection vont chercher dans la mémoire les informations concernant la première cible de référence et tous les ligands connus auxquels elle peut être associée, de manière à permettre l'analyse de toutes les associations entre la seconde cible et les différents ligands extraits ;

* un terminal de service muni d'une interface de communication couplée aux moyens de calcul ou aux moyens de sélection (lorsqu'ils existent) et à un réseau de communication public (de type Internet) ou privé (de type Intranet), et capable de recevoir d'un terminal d'interrogation des données représentatives de la cible sélectionnée et/ou des données représentatives du site récepteur sélectionné et/ou des données représentatives du ligand sélectionné. Bien entendu, lorsque le dispositif ne comprend pas de moyens de sélection, il est préférable que le(s) terminal(aux) d'interrogation adresse(nt) directement les informations représentatives d'une cible et/ou d'un ligand. Lorsque la cible est un acide nucléique ou une protéine, il est avantageux qu'elle soit transmise sous la forme d'une séquence (génotype) et/ou de sa fonction (phénotype). Dans ce cas, il est avantageux que la mémoire puisse également stocker, sous la forme d'une deuxième table, des cibles en correspondance de leur génotype et/ou de leur phénotype, ces cibles étant de préférence identiques au moins aux premières cibles contenues dans la première table. Les moyens de sélection sont alors agencés de manière à déterminer dans la deuxième table la cible sélectionnée lorsqu'ils reçoivent les informations représentatives de sa séquence (génotype) et/ou de son expression et de la fonction de celle-ci (phénotype). Par ailleurs, le terminal de service (qui peut définir un site Internet (ou site « web »)) est de préférence agencé de manière à permettre la transmission au terminal d'interrogation, via le réseau de communication, de la donnée représentative du niveau d'interaction (ou d'association) cible/ligand. De la sorte, il est possible d'obtenir très rapidement, à partir d'un terminal d'interrogation, des données d'affinité qui vont permettre d'aider à choisir un ligand (par exemple un médicament) adapté à la cible d'intérêt ;

* les moyens de calcul et les moyens de comparaison peuvent en outre être agencés de manière à stocker dans les multiplets de la mémoire les valeurs d'affinité ainsi qu'éventuellement les données d'affinité au fur et à mesure de leur détermination, en correspondance des cible et ligand associés. Dans ce cas, les moyens de calcul et/ou les moyens de sélection (lorsqu'ils existent) sont avantageusement capables, en présence d'une cible et d'un ligand, de déterminer dans les multiplets s'il leur correspond une valeur d'affinité, et, dans l'affirmative, d'extraire cette valeur d'affinité pour la transmettre directement aux moyens de comparaison. Cette procédure permet de gagner du temps puisqu'elle évite que l'on refasse des calculs précédemment effectués ;

* les moyens de calcul et/ou les moyens de sélection (lorsqu'ils existent) peuvent également être agencés de manière à fonctionner en mode de balayage, c'est-à-dire, consécutivement à la réception des informations représentatives d'une cible, à déterminer dans la seconde table tous les ligands qui lui sont associés (ou bien tous les ligands qui sont associés à la cible de référence dont la cible reçu constitue une modification), puis à déterminer pour chaque ligand associé sa valeur d'affinité avec la cible reçue ;

* un module de calcul statistique permettant de modifier la valeur d'un paramètre d'affinité ou de réfrence stocké dans la mémoire, par exemple par moyennage statistique, lorsqu'il reçoit d'un utilisateur une donnée, de préférence expérimentale, de paramètre de référence qui diffère du paramètre de référence stocké.

L'invention concerne également un procédé de détermination d'une donnée d'affinité (ou d'association) entre un ligand et une cible, dans lequel on prévoit les étapes suivantes :

a) sélectionner une cible ainsi qu'au moins un ligand susceptible d'interagir avec la cible en l'un de ses sites récepteurs,
b) déterminer une valeur d'affinité représentative de l'énergie d'association entre la cible et le ligand, et
c) délivrer une donnée représentative d'un niveau d'affinité (ou d'association) entre la cible et le ligand à partir de la valeur d'affinité cible/ligand et d'un paramètre de référence choisi.

Le procédé pourra comporter d'autres caractéristiques prises séparément ou en combinaison, et notamment :

* à l'étape a) on peut sélectionner la cible parmi une pluralité de multiplets comportant au moins une information représentative d'une cible, une information représentative d'un ligand et un paramètre de référence pour l'association cible/ligand ;

* à l'étape c) (ou bien en fin d'étape b)), après avoir reçu (ou déterminé) une valeur d'affinité cible/ligand, on extrait de la mémoire le paramètre de référence qui correspond à l'association cible/ligand en vue d'effectuer la comparaison ;

* dans une étape préalable, notamment, à l'étape a), on peut stocker une première table établissant des correspondances entre des premières cibles, dites de référence, et des listes de secondes cibles qui résultent de modifications subies par les premières cibles de référence. Dans ce cas, il est possible de sélectionner lors de l'étape a) une seconde cible et un ligand, susceptible d'interagir avec cette seconde cible, puis d'extraire de la première table la première cible qui est associée à cette seconde cible. Puis, lors de l'étape b) on détermine des première et seconde valeurs d'affinité représentatives des énergies d'association respectivement entre la première cible et le ligand et entre la seconde cible et le ligand, puis on soustrait la première valeur d'affinité de la seconde valeur d'affinité. Enfin, lors de l'étape c) on délivre la donnée représentative du niveau d'affinité entre la seconde cible et le ligand à partir du résultat de la soustraction et du paramètre de référence qui est, de préférence, le paramètre d'affinité entre la première cible extraite et le ligand. Préférentiellement, lors de cette étape c), on applique au résultat de la soustraction une fonction d'affinité de manière à délivrer un paramètre d'affinité, puis on compare ce paramètre d'affinité au paramètre de référence choisi pour déterminer la donnée d'affinité ;

* à l'étape a), la cible et/ou le ligand peuvent être transmis par un terminal d'interrogation via un réseau de communication public ou privé. Dans ce cas, il est avantageux que la cible soit adressée sous la forme d'un génotype et/ou d'un phénotype, et que l'on détermine la cible qui correspond au génotype et/ou au phénotype par interrogation d'une deuxième table établissant des correspondances entre des cibles et leur génotype et/ou phénotype ;

* à l'étape a) la cible et/ou le ligand peuvent être fournis par un terminal d'interrogation via un réseau de communication choisi parmi les réseaux publics et les réseaux privés. Dans ce cas, il est avantageux de prévoir une étape d) dans laquelle on transmet au terminal d'interrogation, via le réseau de communication, la donnée représentative du niveau d'affinité (ou d'association) cible/ligand ;

* à l'étape b) on peut stocker dans les multiplets chaque valeur d'affinité déterminée, en correspondance des cible et ligand associés, de manière à constituer une base de données. Dans ce cas, on prévoit à l'étape a) une phase préliminaire destinée à vérifier dans la quatrième table s'il existe une valeur d'association stockée en correspondance des cible et ligand sélectionnés, de sorte que l'on puisse passer directement à la comparaison de l'étape c) ;

* à l'étape b), à réception d'une cible sélectionnée, on peut également effectuer une interrogation de la première table pour extraire, éventuellement, la première cible de référence qui lui est associée, puis une interrogation de la seconde table pour extraire tous les ligands correspondants à cette première cible et/ou aux secondes cibles associées déjà connues, et enfin déterminer pour chaque ligand extrait sa valeur d'affinité (ou d'association) avec la cible. On passe ensuite à l'étape c).

De nombreuses applications peuvent être envisagées pour les dispositifs et procédés présentés ci-avant, et notamment celles dans lesquelles les cibles sont des cellules infectées, des molécules ou des agents pathogènes. Plus précisément, l'invention est particulièrement intéressante lorsque les agents pathogènes sont des virus tels que le virus d'immuno-déficience humaine de type 1 (VIH-1) et le virus de l'hépatite B (VHB), lorsque les molécules sont des protéines, des acides nucléiques ou des composés de ces derniers, ou encore des PNA, et lorsque les sites récepteurs sont des sites mutés. Dans ces cas particuliers, les ligands sont de préférence des molécules chimiques de synthèse, des macro-molécules biologiques ou des combinaisons de celles-ci. Mais, l'invention concerne également les autres agents microbiens tels que les parasites, les champignons, les autres virus, les bactéries, les agents transmissibles non conventionnels (ATNC), comme par exemple le prion, et tous les ligands dirigés contre ces cibles, ainsi que tout agent étranger à un organisme et devant faire l'objet de traitement avec des molécules.

D'autres caractéristiques et avantages de l'invention apparaîtront à l'examen de la description détaillée ci-après, et des dessins annexés, sur lesquels :

- la figure 1 est un schéma illustrant un dispositif selon l'invention intégré dans un réseau de communication de type Internet ;
- la figure 2 est un schéma, de type diagramme-bloc, illustrant les principaux constituants d'un dispositif selon l'invention ; et
- la figure 3 est un schéma illustrant les principales étapes d'un procédé selon l'invention.

Les dessins annexés sont, pour l'essentiel, de caractère certain. En conséquence, ils pourront non seulement servir à compléter l'invention, mais aussi contribuer à sa définition, le cas échéant.

Dans la description qui suit, il sera fait référence à un dispositif et un procédé destinés à déterminer des données d'affinité entre des cibles et des ligands. Plus précisément, il sera fait référence, seulement à titre d'exemple, à une cible telle que la protéase du virus d'immunodéficience humaine (VIH-1) et à des ligands susceptibles d'inhiber la protéase du VIH-1 par association, de manière à bloquer la phase tardive de la maturation virale.

L'invention propose tout d'abord un dispositif qui, de préférence, comprend un terminal informatique tel qu'un serveur ou un ordinateur individuel.

En variante, le dispositif selon l'invention peut ne pas comprendre de terminal informatique, mais être implanté ou destiné à être implanté dans un terminal informatique. Dans ce cas, il peut être intégralement réalisé sous la forme de modules logiciels (ou programmes) implantés, ou destinés à l'être, dans un disque dur d'ordinateur, fixe ou amovible, une disquette ou plus généralement sur tout type de support d'informations susceptible de coopérer avec un ordinateur éventuellement via un lecteur interne ou externe.

Le dispositif peut donc être réalisé soit sous la forme d'un logiciel qui, une fois intégré dans un calculateur (ou ordinateur), permet audit calculateur de délivrer des données d'affinité entre une cible et au moins un ligand, soit sous la forme d'une combinaison de circuits électroniques (« hardware »), par exemple arrangés sur une carte électronique dédiée, et d'instructions informatiques arrangées en modules logiciels (« software »), soit encore sous la forme d'un terminal informatique individuel ou d'un terminal de service raccordé à un réseau public ou privé, et équipé du logiciel précité ou de la combinaison (hardware/software) précitée.

**EP 1 209 610 A1**

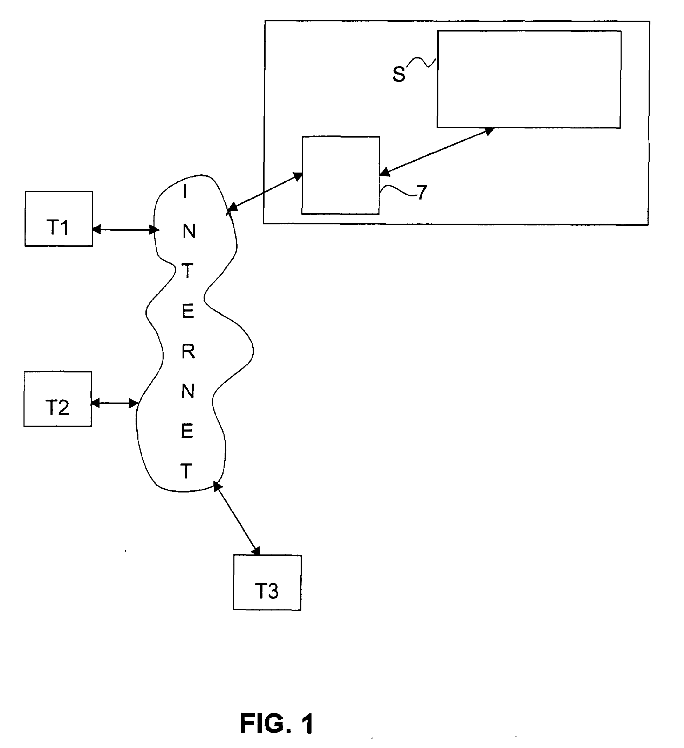

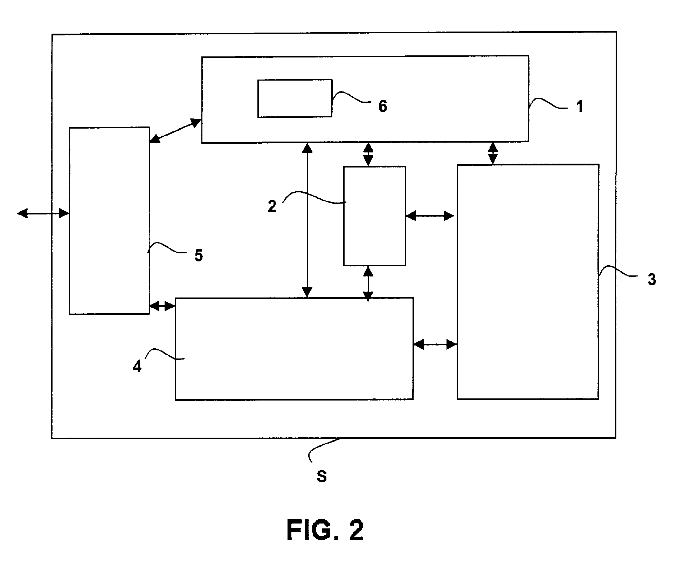

Dans l'exemple illustré sur les figures 1 et 2, le terminal informatique est un serveur S qui définit un site Internet dédié, notamment, au calcul de données d'affinité cible/ligand. Ce serveur S est donc raccordé à un réseau public (Internet). Mais, en variante, le serveur pourrait être raccordé à un réseau privé de type Intranet.

Ce serveur S comporte, tout d'abord, un module de calcul 1 permettant de modéliser (ou calculer) la conformation structurale et énergétique d'une cible ou d'un ligand.

Il est bien connu que les (macro-)molécules, notamment, se caractérisent par leurs différences structurales, et plus particulièrement leurs différences de forme, de distribution de charge électronique et de distribution de charge nucléique. Plus précisément, ces distributions de charge nucléique et électronique contribuent fortement aux interactions électrostatiques à longue portée qui régissent les associations entre une cible et un ligand, en un site préférentiel.

De nombreux modèles ont été développés pour déterminer l'énergie libre d'une cible au niveau de l'un de ses sites, et plus précisément les minima d'énergie libre qui permettent de quantifier le degré de stabilité d'un « système ».

Le module de calcul 1 du dispositif selon l'invention est agencé pour effectuer de telles minimisations énergétiques (par exemple en suivant les lois de la dynamique moléculaire), de manière à déterminer la configuration optimale d'une cible telle que la protéase du VIH-1 (ou plus généralement une molécule ou une macromolécule), à partir de sa composition moléculaire. La cible modélisée est donc celle qui possède la configuration énergétique la plus probable, c'est-à-dire minimale, parmi toutes les configurations possibles.

Un certain nombre de logiciels de calcul, bien connus de l'homme de l'art, permettent ce type de modélisation. On citera, notamment, les logiciels MSI, WOOLFORD, MODELLER (ou LIGPLOT) et GENMOL.

Plus précisément, le logiciel WOOLFORD, décrit dans le document WO98/54665, permet de déterminer par le calcul les emplacements des sites d'une cible et les énergies libres associées à ces sites. La confrontation des énergies libres des différents sites permet ensuite de sélectionner ceux qui possèdent les énergies libres les plus faibles et qui par conséquent sont censés conférer les meilleures stabilités en cas d'association à un ligand.

Le logiciel GENMOL est conçu de manière à déterminer la structure la plus probable d'une cible (telle qu'une (macro-)molécule) à partir de ses seuls constituants, y compris dans le désordre, et la configuration la plus probable d'une entité constituée par association d'une cible et d'un ligand. Ce logiciel de modélisation peut être obtenu sur le site web dont l'adresse est http:// chimir1.univ-mrs.fr, ou bien directement au Laboratoire de Chimie et Matériaux Organiques, Modélisation (LCMOM) - Faculté des Sciences de Luminy - Case 901 - 163, Avenue de Luminy - 13288 MARSEILLE CEDEX 09.

Le module de calcul 1 selon l'invention est en outre capable d'extraire des modélisations d'une cible, d'un ligand et d'une entité cible/ligand, leurs énergies libres, pour en déduire une valeur d'affinité représentative de l'énergie d'association de l'entité cible/ligand.

Comme indiqué dans l'introduction, on entend par association tout type d'interaction directe ou indirecte entre une cible et un ligand, au niveau d'un site récepteur principal de la cible (d'autres interactions « secondaires » avec des sites auxiliaires de cette cible peuvent également intervenir dans l'association). En d'autres termes, une association n'implique pas obligatoirement une ou plusieurs liaisons entre le ligand et les résidus de la cible.

Préférentiellement, la valeur d'affinité calculée (ou déterminée) par le module de calcul 1, à partir de la connaissance d'informations représentatives d'une cible et d'un ligand, est l'énergie d'association entre cette cible et ce ligand. Mais, il pourrait s'agir de toute autre valeur représentative de l'énergie d'association, c'est-à-dire liée à celle-ci par une équation ou déductible de celle-ci par des opérations mathématiques.

Comme le sait l'homme de l'art, il découle de l'équation de H.Eyring que la vitesse d'association d'une cible avec un ligand est liée à la barrière énergétique qu'il faut franchir pour conduire à l'association cible/ligand. Cette barrière énergétique est l'énergie d'association $E_A$ cible/ligand (ici, la valeur d'affinité).

Par ailleurs, il découle de la statistique de Boltzmann que la durée de vie d'une entité cible/ligand, c'est-à-dire la durée pendant laquelle la cible interagit avec le ligand (ou durée d'interaction, ou encore durée d'association), est liée à la vitesse d'association cible/ligand. Il en résulte que la durée de vie de l'entité cible/ligand est directement liée à l'énergie d'association cible/ligand. A l'équilibre thermodynamique on a une relation du type de celle donnée ci-après :

$$\log (D_{\text{association mutation}} / D_{\text{association référence}}) \propto (E_{A0} - E_{A1}) / RT = \Delta E / RT,$$

où $D_{\text{associauon référence}}$ est un paramètre d'affinité représentant la durée d'association entre un ligand et une cible de référence généralement non mutée, $D_{\text{association mutation}}$ est un paramètre d'affinité représentant la durée d'association entre le ligand et une mutation de la cible de référence (ou cible mutée), $E_{A0}$ représente l'énergie d'association de l'entité cible de référence/ligand et $E_{A1}$ représente l'énergie d'association de l'entité cible mutée/ligand, T est la température et R la constante des gaz parfaits.

Préférentiellement, les moyens de calcul 1 déterminent l'énergie d'association entre une cible et un ligand à partir du calcul de l'énergie libre de cette cible et de l'énergie libre de l'association de cette cible avec le ligand. Par convention, les énergies d'association sont données en kcal/mole et présentent des valeurs négatives, de sorte que l'énergie libre d'une entité cible/ligand soit égale à l'énergie libre de la cible additionnée à l'énergie d'association cible/ ligand (laquelle est alors une valeur négative).

A titre d'exemple, l'énergie d'association entre la protéase du VIH-1 non mutée (ici, la cible de référence) et le ritonavir, calculée à l'aide d'un module de calcul 1 utilisant le logiciel GENMOL, est égale à environ -92,1 kcal/mole, tandis que pour les mutations M46I, I84V, V82A, V82T et V82F on obtient des énergies d'association avec le ritonavir respectivement d'environ -92 kcal/mole, -90,2 kcal/mole, -89,1 kcal/mole, -89,7 kcal/mole et -87,8 kcal/mole. Ces valeurs ne constituent que des exemples. Toutes les énergies d'association entre le ritonavir (ou tout autre ligand) et d'autres mutations de la protéase du VIH-1 peuvent être calculées, y compris lorsque la mutation concerne plusieurs résidus. Par exemple, pour les mutations G84V + L101, V82A + I54V + M46I + A71V et 154V + V82A on obtient des énergies d'association avec le ritonavir respectivement d'environ -93,2 kcal/mole, -89,4 kcal/mole et -89,2 kcal/mole.

Egalement à titre d'exemple, l'énergie d'association entre la protéase du VIH-1 non mutée (ici, la cible de référence) et le nelfinavir, calculée à l'aide d'un module de calcul 1 utilisant le logiciel GENMOL, est égale à environ -81,4 kcal/mole, tandis que pour les combinaisons de mutations suivantes (D30N + L10I + L63LI + A71T + I93LI13V + I15V + 162V), (D30N + N88D), (D30N + M361 + L63P + N88DI13V + E35D), (D30N + L10F + L63P + V77I + N88DK14Z + I15V + R41K) on obtient des énergies d'association avec le nelfinavir respectivement d'environ -81,8 kcal/mole, -81,3 kcal/mole, -79,5 kcal/mole et -78,5 kcal/mole.

Il est important de noter que les valeurs calculées de l'énergie d'association sont données avec une incertitude d'environ plus ou moins 0,3 kcal/mol.

De la relation donnée ci-avant, on déduit le rapport entre les durées d'association d'une cible mutée et de la cible de référence dont elle est issue:

$$D_{association\ mutation} / D_{association\ référence} \propto 10^{\Delta E/RT}.$$

Ce rapport montre que de faibles écarts entre les énergies d'association cible de référence/ligand et cible mutée/ligand peuvent entraîner de grandes différences entre les durées d'association cible de référence/ligand et cible mutée/ligand.

Ainsi, pour une différence d'énergie d'association $\Delta E$ (= $E_{A0}$ - $E_{A1}$) d'environ -5 kcal/mole, on obtient un rapport de durée d'association entre une entité mutée, constituée d'une cible mutée et d'un ligand, et une entité de référence, constituée d'une cible de référence (non mutée) et de ce même ligand, de l'ordre de $10^5$. Par conséquent, la durée d'association de la cible mutée avec le ligand est $10^{\Delta E/RT}$ fois celle de l'association de la cible de référence avec ce ligand, soit ici de l'ordre de $10^5$ fois plus petite.

Ainsi, si l'on connaît la valeur de la durée (ou vitesse) d'association cible de référence/ligand, on peut déterminer la valeur de la durée d'association cible mutée/ligand. Cette durée d'association entre la cible de référence et le ligand peut être obtenue de façon expérimentale. Bien entendu, certaines durées d'association peuvent être également connues pour des entités cible mutée/ligand.

La durée d'association (comme la vitesse d'association) est donc un paramètre d'affinité permettant de quantifier l'aptitude d'une cible à s'associer avec un ligand. Mais, il existe d'autres paramètres d'affinité équivalent à la durée d'association, comme notamment la résistance d'une cible vis-à-vis d'un ligand. C'est d'ailleurs ce dernier paramètre qui est le plus souvent connu pour les cibles de référence. Il est obtenu lors de tests expérimentaux effectués *in vivo* ou *in vitro.*

La résistance peut être liée à $\Delta E$ par une relation empirique du type log R = A . $\Delta E$ + B, A et B étant des constantes dépendant du ligand. Cette relation est obtenue pour un ligand donné à partir de tests d'association effectués sur quelques mutations d'une cible de référence, et du calcul pour chacune de ces associations mutation/ligand du $\Delta E$ correspondant.

Dans le tableau ci-dessous se trouvent mentionnées, à titre d'exemple, la résistance (connue) et l'énergie d'association (calculée) de protéases du VIH, non-mutée (wild type) et mutées, vis-à-vis du ritonavir :

| | Résistance | Energie d'association (Kcal/mole) |
|---|---|---|
| Protéase non mutée | 0,04578 | -91,1 |
| M46I | 1 | -92 |
| I84V | 7 | -90,2 |
| V82A | 12 | -89,1 |
| V82T | 30 | -89,7 |
| V82F | 52 | -87,8 |
| G84V + L10I | 3 | -93,2 |
| V82A + I54V + M46I + A71V | > 8 | -89,4 |
| I54V + V82A | > 8 | -89,2 |

Dans ce cas, les constantes A et B de la relation mentionnée ci-avant valent respectivement + 0,4651 et +/- 0,15. En d'autres termes, la résistance R des protéases mutées du VIH vis-à-vis du ritonavir sont liés à l'énergie d'association $\Delta E$ par la relation : log R = 0,4651 . $\Delta E$ +/- 0,15.

Dans le tableau ci-dessous se trouvent mentionnés, à titre d'exemple, la résistance (connue) et l'énergie d'association (calculée) de protéases du VIH, non-mutée (wild type) et mutées, vis-à-vis du nelfinavir :

| | Résistance | Energie d'association (Kcal/mole) |
|---|---|---|
| Protéase non mutée | 0,001 | -81,4 |
| D30N + L10I + L63LI + A71T + I93LI13V + I15V + I62V | 5 | -81,8 |
| D30N + N88D | 7 | -81,3 |
| D30N + M36I + L63P + N88DI13V + E35D | 23 | -79,5 |
| D30N + L10F + L63P + V77I + N88DK14Z + I15V + R41K | 77 | -78,5 |

Dans ce cas, les constantes A et B de la relation mentionnée ci-avant valent respectivement + 0,3468 et + 29,036. En d'autres termes, la résistance R des protéases mutées du VIH vis-à-vis du nelfinavir est liée à leur énergie d'association $\Delta E$ par la relation : log R = 0,3468 . $\Delta E$ + 29,036.

Il est important de noter que les valeurs des résistances mentionnées ci-dessus sont issues de phénotypages publiés dans des revues scientifiques internationales.

Connaissant la relation qui lie la résistance à $\Delta E$ pour certaines mutations issues d'une même cible de référence, on peut alors l'appliquer aux autres mutations de cette cible de référence. Il est alors possible d'estimer la résistance de chaque mutation d'une cible de référence donnée vis-à-vis d'un ligand donné.

Mais le paramètre d'affinité déterminé, par exemple la résistance d'une mutation vis-à-vis d'un ligand, peut être une information difficile à exploiter. Par conséquent, selon l'invention, une fois déterminé le paramètre d'affinité entre une cible et un ligand, on effectue préférentiellement une comparaison de ce paramètre d'affinité avec un paramètre de référence. Par exemple, dans le cas de la résistance d'une mutation vis-à-vis d'une molécule-ligand, le paramètre de référence servant à la comparaison est préférentiellement la résistance connue de la cible de référence, dont elle est issue, vis-à-vis du ligand

La comparaison peut consister en une opération, par exemple de type soustraction ou rapport (mais des opérations plus complexes peuvent être envisagées), dont le résultat est de nature à indiquer si l'affinité cible/ligand analysée est ou non de qualité.

Par exemple, on peut arbitrairement décider qu'une résistance égale à 1 (un) désigne une association cible de référence/ligand durable, c'est-à-dire efficace dans le temps, et donc toute valeur de résistance inférieure à 1 (un) désigne une association encore plus durable. Dans ce cas, si la comparaison consiste à effectuer le rapport entre la résistance de la mutation sur la résistance de la cible de référence, alors on peut considérer que tout résultat de comparaison strictement supérieur à 1 (un) indique une mauvaise association ou une résistance forte, tandis qu'un résultat de comparaison strictement inférieur à 1 (un) indique une très bonne association ou une résistance très faible.

La comparaison est effectuée par un module de comparaison 3 couplé au module de calcul 1, réalisé sous la forme d'un module-logiciel (software) et/ou de composants électroniques (hardware). Et le résultat de cette comparaison est communiqué sous la forme d'une donnée d'affinité de type message ou valeur numérique.

Le message est de préférence un avertissement du type :

i) « mauvaise association » ou « résistance probable». Un tel message correspond à un résultat de comparaison qui, dans l'exemple précité, est strictement supérieur à 1 ;

ii) « très bonne association » ou « très faible résistance ». Un tel message correspond à un résultat de comparaison qui, dans l'exemple précité, est strictement inférieur à 1 ;

iii) « bonne association» ou « faible résistance». Un tel message correspond à un résultat de comparaison qui, dans l'exemple précité, est de l'ordre de 1.

D'autres types de message, plus précis, peuvent être envisagés. Ainsi, le message peut indiquer si le niveau de résistance est faible, moyen ou élevé, par exemple en référence à un seuil.

Ces messages, ou d'autres messages équivalents, peuvent être affichés sur le moniteur de l'utilisateur interrogateur ou imprimés sur son imprimante.

La donnée sous forme d'une valeur numérique est le résultat brut de l'opération de comparaison. Elle peut ensuite être comparée par l'utilisateur à des valeurs stockées dans une table en correspondance d'un message, du type de ceux indiqués ci-dessus, éventuellement complétées par d'autres informations ou avertissements.

En variante, on peut accompagner le paramètre de référence associé à une entité cible/ligand d'une information complémentaire. Par exemple, l'information complémentaire pourra porter sur une valeur limite de résistance au-delà de laquelle la résistance cible/ligand est considérée comme rédhibitoire. Dans ce cas, un résultat inférieur à la valeur limite indique que l'interaction entre la cible mutée et le ligand a une bonne probabilité d'être de qualité, tandis qu'un résultat strictement supérieur à cette valeur limite indique que l'interaction entre la cible mutée et le ligand a une forte probabilité d'être de mauvaise qualité, voire impossible.

Dans une autre variante, le dispositif comporte dans ses moyens de mémorisation une table de correspondance entre des valeurs et des messages, si bien qu'une fois que le résultat de l'opération de comparaison est connu, le module de comparaison extrait de cette table le message (et éventuellement les informations complémentaires associées) correspondant à la valeur du résultat de comparaison et l'affiche sur le moniteur de l'utilisateur interrogateur ou l'imprime sur son imprimante.

En lisant la donnée de comparaison, un praticien sait immédiatement s'il peut, ou non, utiliser un ligand pour inhiber une cible.

Le paramètre de référence pourra également être un intervalle de valeurs. Cela peut s'avérer préférable, notamment lorsque le paramètre d'affinité ou de référence résulte d'un faible nombre de tests expérimentaux et que de ce fait la statistique est peu fiable. C'est notamment le cas de la protéase du VIH-1, de la transcriptase inverse du VIH-1, et d'un virus tel que celui de l'hépatite B (VHB), pour lesquels les paramètres d'affinité et de référence résultent de tests peu nombreux effectués *in vivo* sur des patients malades, ou bien *in vitro.*

Pour d'autres applications, comme notamment le criblage à haut débit, les paramètres d'affinité et de référence résultent de tests *in vitro* reproductibles à grande échelle et par conséquent permettant de fournir des valeurs fiables.

Lorsque plusieurs entités cible/ligand ont été initialement sélectionnées par un opérateur, le dispositif délivre autant de données représentatives d'un niveau d'affinité qu'il y a d'entités, ces données étant accompagnées d'informations désignant la cible et le ligand associés.

Les paramètres d'affinité et de référence, fournis par un opérateur ou calculés par le module de calcul 1, sont stockés dans des moyens de mémorisation, de préférence dans des multiplets qui comportent chacun au moins une donnée représentative d'une cible et une donnée représentative d'un ligand. Préférentiellement, les moyens de mémorisation comprennent une mémoire 2 dans laquelle sont stockés les multiplets, sous la forme d'un fichier, ou bien dans un tableau d'une bibliothèque du dispositif.

Préférentiellement, dès qu'une différence d'énergie d'association (ΔE) est calculée par le module de calcul 1, celle-ci est également stockée dans le multiplet correspondant. D'autres données peuvent être également stockées dans les multiplets dès qu'elles sont calculées, ou connues. On peut citer notamment, l'énergie d'association de la cible avec le ligand, ce qui permet de gagner du temps lors du calcul de la différence d'énergie d'association (ΔE).

Bien entendu, lorsque pour une entité cible mutée/ligand on dispose d'un paramètre de référence issu de tests expérimentaux, c'est ce paramètre de référence expérimental qui est utilisé et stocké dans le multiplet.

Il est clair que l'invention n'est pas limitée aux modes de comparaison présentés ci-avant, à titre d'exemple. Tout autre type de comparaison reposant sur les valeurs d'affinité, paramètres d'affinité et paramètre de référence peut être envisagé dans le cadre de cette invention.

Les moyens de calcul 1 et les moyens de comparaison 3 peuvent être intégralement réalisés sous la forme de modules logiciels (ou programmes) stockés sur un disque dur du terminal informatique, interne ou externe, ou bien sur une disquette ou plus généralement sur tout type de support d'informations susceptible de coopérer avec un ordinateur éventuellement via un lecteur interne ou externe.

De préférence, les moyens de mémorisation stockent une première table qui établit une correspondance entre des cibles de référence et leurs mutations connues (appelées cibles mutées). Par ailleurs, les moyens de mémorisation pourront également stocker, préférentiellement, une seconde table établissant une correspondance entre des cibles (qu'il s'agisse de cibles de référence ou de cibles mutées) et des ligands avec lesquels ils sont susceptibles d'interagir.

De la sorte, lorsque le dispositif ne dispose que d'informations sur une cible mutée à analyser, sans référence à un ligand, on peut déterminer dans les tables de correspondance et les multiplets le ou les ligands susceptible(s) d'interagir avec la cible reçue. Trois cas peuvent survenir. Dans un premier cas, il existe un ou plusieurs multiplet(s) qui désigne(nt) la cible reçue et un ou plusieurs ligands respectivement. Dans ce cas, les ligands sont immédiatement extraits et transmis aux module de calcul 1. Dans le second cas, il n'existe pas de multiplet qui désigne la cible reçue, mais la cible est répertoriée dans la première table de correspondance. Il faut alors chercher dans cette première table de correspondance, la cible de référence qui est associée à la cible reçue, c'est-à-dire celle dont elle constitue une mutation, puis on cherche dans la seconde table et dans les multiplets les ligands qui sont associés à cette cible de référence. Dans le troisième cas, il n'existe pas de multiplet qui désigne la cible reçue et la cible n'est pas répertoriée dans la première table de correspondance. Il faut alors que le module de calcul détermine par modélisation la constitution structurale et énergétique de la cible reçue, puis détermine par comparaison, avec les contenus des tables et multiplets, la cible de référence qui présente la constitution la plus proche de celle de la cible reçue.

Toutes ces déterminations de cibles de référence et de ligands peuvent être effectuées par un module de sélection 4, de préférence couplé à l'interface de communication 5 du terminal informatique et au module de calcul 1. De la sorte, lorsque le dispositif reçoit des données désignant une cible, le module de sélection 4 se charge de déterminer, d'une part, les informations représentatives de cette cible, et d'autre part, un ou plusieurs ligands susceptibles d'interagir avec la cible reçue ainsi qu'éventuellement la cible de référence associée à la cible reçue. Une fois en possession de ces informations, le module de sélection 4 peut les transmettre au module de calcul 1 pour qu'il détermine une ou plusieurs valeurs d'affinité. Par ailleurs, lorsque le module de sélection 4 détermine un multiplet qui correspond à la cible reçue, il en extrait directement les données pour les transmettre soit directement au module de comparaison 3 lorsqu'elles comportent la valeur d'affinité et le paramètre d'affinité associé, de sorte qu'il effectue sa comparaison, soit au module de calcul 1 lorsqu'elles ne comportent que le paramètre d'affinité, de sorte qu'il effectue sa modélisation de l'entité puis détermine la valeur d'affinité.

Préférentiellement, le module de sélection 4 est également agencé pour proposer à l'utilisateur du terminal informatique d'effectuer sa sélection de cible et/ou ligand(s) parmi des listes affichées sur le moniteur et préférentiellement mémorisées dans la mémoire 3 du dispositif. Il peut s'agir directement des tables de correspondance. En variante, cette sélection peut être gérée par un module auxiliaire couplé aux moyens de mémorisation et au module de calcul 1 et/ou au module de sélection 4. Ce cas est particulièrement avantageux en matière de temps de traitement, étant donné que toutes les données qui caractérisent une cible et un ligand offerts à la sélection sont connues du dispositif selon l'invention, ce qui lui évite d'avoir à les déterminer, par exemple lors d'une modélisation complète.

Lorsque l'utilisateur décide de ne pas fonctionner en mode de sélection, ou lorsque cette option n'est pas offerte par le dispositif, il doit fournir au dispositif selon l'invention des données caractérisant une cible, ainsi qu'éventuellement un ou plusieurs ligands. Ces données peuvent comporter suffisamment d'informations pour permettre au module de calcul 1 de déterminer une ou plusieurs valeurs d'affinité pour chaque association cible/ligand correspondant à la requête de l'utilisateur. Lorsque ces données sont insuffisantes, le module de sélection 4 doit déterminer les données représentatives de la cible et du ou des ligands susceptibles d'interagir avec cette cible parmi les informations stockées dans les tables et multiplets des moyens de mémorisation. Bien entendu, lorsque ces informations ne sont pas disponibles, le module de calcul 1 procède à leur détermination en effectuant une modélisation complète.

La sélection peut s'effectuer à distance lorsque le dispositif selon l'invention est implanté dans un terminal de service S (ou site) auquel le terminal de l'utilisateur est raccordé via un réseau public (de type internet) ou un réseau privé (de type intranet). Dans ce cas, l'utilisateur doit tout d'abord se connecter au site (et plus précisément au terminal de service), puis transmettre à ce site, par l'intermédiaire de son propre terminal d'utilisateur, les données représentatives d'une cible et éventuellement d'un ou plusieurs ligands, de manière à obtenir une ou plusieurs données d'affinité en retour.

Dans le cas de la protéase du VIH-1, il est avantageux que l'utilisateur transmette, soit à son terminal d'interrogation, soit au terminal de service, les données représentatives de la cible sous la forme d'un génotype (par exemple une suite de mutations) et/ou d'un phénotype (par exemple une observation clinique).

A cet effet, le dispositif selon l'invention comporte, de préférence, une troisième table établissant la correspondance entre des cibles, préférentiellement celles qui sont déjà stockées dans les première et seconde tables et multiplets, et leur génotype et/ou phénotype. En effet, cela permet au dispositif, à réception des données de génotype et/ou phénotype, d'obtenir, par interrogation de la troisième table de correspondance, les informations représentatives de la cible et de les transmettre au module de calcul 1.

Grâce à l'invention, il est donc désormais possible à un utilisateur, comme par exemple un médecin, d'adresser, de préférence sous une forme confidentielle, par exemple cryptée, les données représentatives d'une cible caractérisant son patient, de manière à obtenir en retour, soit de son terminal d'interrogation, soit du terminal de service, une donnée d'affinité caractérisant l'aptitude de la cible, objet de l'interrogation, à interagir avec un ou plusieurs ligands connus. Un médecin peut ainsi savoir très rapidement si un médicament connu (ligand) est susceptible d'interagir efficacement avec la cible de son patient. Ce médecin peut alors choisir le médicament (ou ligand) qui lui paraît le plus approprié au cas de son patient.

Mais, on peut également se connecter au site (Internet) pour obtenir des données d'affinité pour des séries de cibles appartenant à une même « famille », par exemple les mutations d'une cible de référence. Ainsi, un utilisateur peut adresser au dispositif selon l'invention une protéase donnée (ou une cible), de sorte qu'il détermine la liste des mutations de cette protéase et, pour chacune de ces mutations, l'inhibiteur (ou ligand) avec lequel elle présente le niveau d'affinité le plus élevé. En d'autres termes, le dispositif détermine pour chaque mutation et chaque ligand une donnée d'affinité, puis le ligand avec lequel elle présente la plus forte affinité. Ensuite, le dispositif élabore un tableau comparatif présentant chaque mutation avec le ligand avec lequel elle présente la plus forte affinité.

Le dispositif selon l'invention peut également être utilisé pour déterminer plusieurs données d'affinité de types différents pour une même cible et une série de ligands, puis déterminer pour chaque type de donnée d'affinité le ligand qui présente l'affinité la plus forte avec la cible. Le résultat peut ensuite être présenté sous forme d'un tableau.

La même opération peut être effectuée pour chaque mutation d'une cible de référence, telle qu'une protéase, de sorte que l'on puisse comparer dans des tableaux associés à chacun des types de donnée d'affinité les ligands présentant la plus forte affinité avec les différentes mutations.

Les données d'affinité peuvent être complétées par d'autres paramètres relatifs à des propriétés physico-chimiques de l'entité cible/ligand et/ou par d'autres informations, comme par exemple des indications de combinaison du ligand avec un ou plusieurs autres ligands. L'invention peut ainsi permettre de déterminer des combinaisons de ligands (médicaments) capables d'agir efficacement vis-à-vis d'une cible mutée donnée.

Préférentiellement, le dispositif selon l'invention comporte également un module de calcul statistique 6 permettant de modifier la valeur d'un paramètre d'affinité ou de référence mémorisé dans un multiplet. Cette modification peut être automatique, c'est-à-dire sans intervention de l'utilisateur, soit commandée, c'est-à-dire sur ordre de l'utilisateur ou d'un superviseur de site.

Cette modification peut se produire à réception d'une nouvelle valeur de paramètre d'affinité ou de référence. Elle peut consister, selon les instructions adressées par l'utilisateur, soit en un remplacement pur et simple du précédent paramètre d'affinité ou de référence par le nouveau paramètre d'affinité ou de référence, soit en un moyennage statistique destiné à affiner le paramètre précédent. Lorsqu'il s'agit d'affiner le paramètre d'affinité ou de référence stocké dans un multiplet, il est essentiel que le module de calcul statistique 6 connaisse l'historique des valeurs de ce paramètre de manière à pouvoir attribuer un poids statistique approprié au paramètre qui lui est communiqué.

Par ailleurs, il est particulièrement avantageux qu'à chaque fois qu'une nouvelle cible est modélisée et associée à un ligand un multiplet soit créé pour stocker les informations représentatives de cette cible et de ce ligand, la valeur d'affinité cible/ligand déterminée et le paramètre de référence utilisé pour la comparaison. Cela évite de recalculer la valeur d'affinité utilisée et de rechercher le paramètre de référence pertinent, et permet par conséquent de procéder directement, après extraction de la valeur d'affinité et du paramètre de référence, à la comparaison en vue de l'obtention de la donnée d'affinité.

Le module de calcul statistique 6 est de préférence réalisé sous la forme d'un module logiciel (ou programme), éventuellement intégré dans le module de calcul 1, et implanté dans le disque dur d'un terminal, fixe ou amovible, une disquette ou plus généralement sur tout type de support d'informations susceptible de coopérer avec un terminal, éventuellement via un lecteur interne ou externe.

Le dispositif selon l'invention peut également être utilisé pour effectuer des validations de valeurs d'affinité. L'exemple qui est décrit ci-après concerne également l'application aux protéines. On considère que la valeur d'affinité est l'énergie d'association.

Tout d'abord, on extrait de la base de données Protein Data Base (PDB), accessible sur le site Internet dont l'adresse est http//www.rcsb.org, les informations définissant une protéine (appelée P1) et un premier ligand (appelé L1), par exemple la protéase du VIH non mutée associée au ritonavir. De préférence, on extrait des informations structurales obtenues par cristallographie. Puis, on détermine l'énergie d'association $E_A$(P1/L1) de l'entité « native » E1, constituée de l'association entre P1 extrait de PDB et L1 extrait de PDB, à l'aide du module de calcul 1.

Puis, on doit vérifier que les résultats des calculs énergétiques obtenus à partir des informations structurales déterminées par le module de calcul coïncident avec les résultats des calculs énergétiques obtenus à partir des informations structurales issues de la base PDB.

On modélise donc, avec le module de calcul 1, la protéine P1 et le ligand L'1, et l'on détermine l'énergie d'association $E'_A$(P1/L1) de l'entité E1 constituée de l'association entre P1 modélisée et le ligand L1 modélisé.

On compare ensuite $E'_A$(P1/L1) et $E_A$(P1/L1), et l'on stocke $E_A$(P1/L1) dans un multiplet associé à P1 et L1, lorsque $E'_A$(P1/L1) est sensiblement égal à $E_A$(P1/L1). Lorsque $E'_A$(P1/L1) est sensiblement différent de $E_A$(P1/L1) on stocke $E'_A$(P1/L1) dans le multiplet associé à P1 et L1.

On procède de la même façon pour déterminer les énergies d'association entre le ligand L1 et les différentes mutations M1i de la protéase P1.

On modélise donc, avec le module de calcul 1, une première mutation de la protéine P1 (appelée M11), et l'on détermine l'énergie d'association $E_A$(M11/L1) de l'entité mutée E11 constituée de l'association entre la première mutation M11 modélisée et le ligand L1 extrait de PDB.

On extrait ensuite de PDB les informations définissant l'entité mutée E'11, constituée de cette association entre la première mutation M11 et le ligand L1. Puis l'on détermine l'énergie d'association $E'_A$(M11/L1) de l'entité mutée E'11 à l'aide du module de calcul 1.

On compare alors $E'_A$(M11/L1) et $E_A$(M11/L1), et l'on stocke $E_A$(M11/L1) dans un multiplet associé à M11 et L1, lorsque $E'_A$(M11/L1) est sensiblement égal à $E_A$(M11/L1). Lorsque $E'_A$(M11/L1) est sensiblement différent de $E_A$(M11/L1) on stocke $E'_A$(M11/L1) dans le multiplet associé à P1 et L1.

On procède alors de la même façon pour la mutation suivante M12 et le ligand L1, puis pour toutes les mutations M1i de P1 répertoriées dans la base PDB.

On peut ensuite déterminer les énergies d'association de P1 et de ses mutations M1i avec un second ligand L2. Puis, on effectue de même pour une autre protéase P2, et ainsi de suite.

L'invention porte également sur un procédé pour la mise en oeuvre du dispositif présenté ci-avant. Ce procédé comporte au moins les étapes mentionnées ci-après.

On effectue tout d'abord la sélection d'une cible et d'au moins un ligand susceptible d'interagir avec la cible en l'un de ses sites récepteurs. Le mot "sélectionné" doit être compris dans un sens large. Il s'agit en fait de déterminer les informations représentatives d'une cible et d'au moins un ligand qui vont permettre aux moyens de calcul de modéliser chaque entité cible/ligand. Puis, on détermine pour chaque association cible/ligand sélectionnée une valeur d'affinité représentative de l'énergie d'association entre cette cible et ce ligand (il peut s'agir simplement de cette énergie d'association). Cette détermination s'effectue par un calcul lorsque la valeur d'affinité n'est pas préalablement connue, ou par extraction à l'intérieur des multiplets. Enfin, on délivre pour chaque association cible/ligand une donnée représentative d'un niveau d'affinité entre la cible et le ligand, à partir de la valeur d'affinité préalablement déterminée et d'un paramètre de référence choisi. Ce paramètre de référence provient, de préférence, des multiplets. Mais, en variante, il pourrait s'agir d'un paramètre fixe valable pour un même type de cible, par exemple une cible de référence et toutes ses mutations.

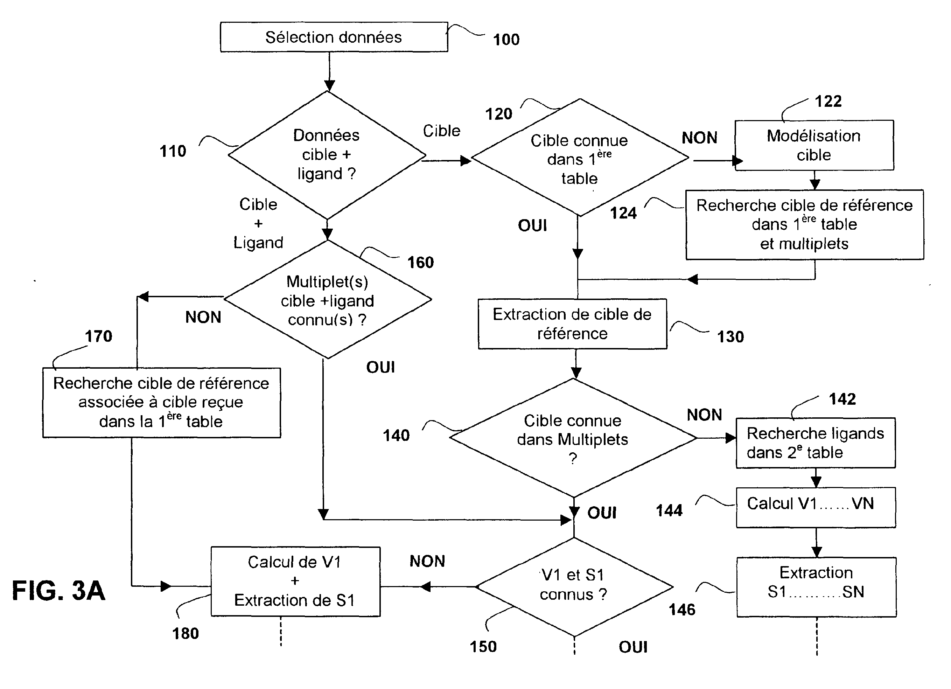

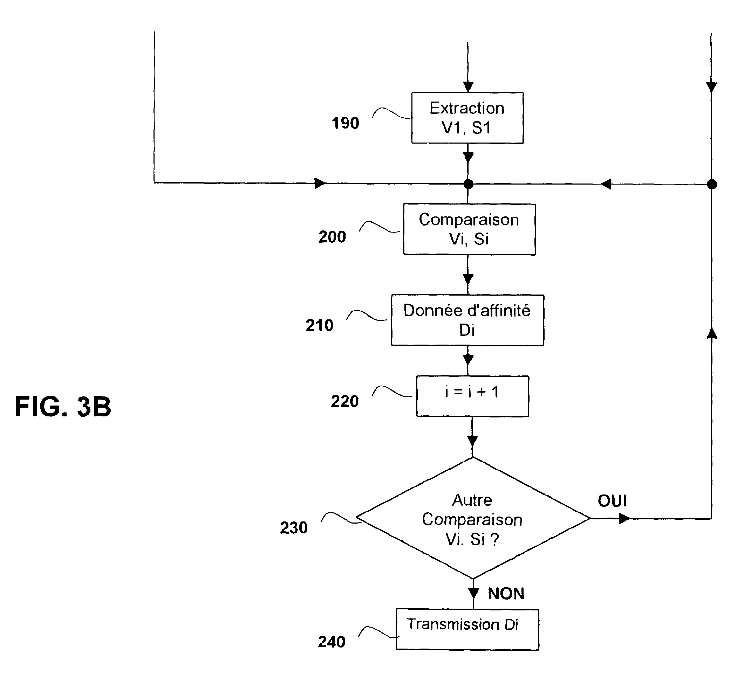

On se réfère maintenant à la figure 3 pour décrire plus en détail un procédé selon l'invention, plus complet.

Dans une étape 100, on sélectionne des données de cible et/ou ligand. Cette sélection peut consister, soit en la fourniture de données représentatives d'une cible seule, soit d'une cible et d'un ou plusieurs ligands. Mais, comme indiqué précédemment, elle peut également consister en la sélection, parmi des listes, d'une cible accompagnée éventuellement d'un ou plusieurs ligands.

Dans une étape 110, on effectue un test destiné à déterminer la nature des données fournies. Plus précisément, on détermine si les données contiennent des informations sur la cible seule, ou sur la cible et un ou plusieurs ligands.

Si le test 110 indique que seule la cible a été désignée, alors on effectue dans une étape 120 un nouveau test destiné à déterminer si cette cible est répertoriée dans la première table de correspondance cibles de référence/ cibles mutées.

Si la cible mutée reçue est répertoriée dans la première table de correspondance, alors on passe à une étape 130 qui sera décrite plus loin. En revanche, si la cible n'est pas répertoriée dans la première table de correspondance, alors on procède dans une étape 122, à la modélisation de la cible de manière à déterminer sa configuration tant sur le plan structural qu'énergétique (phase de modélisation). Puis, dans une étape 124, on recherche dans la première table de correspondance et dans les multiplets, la cible de référence qui correspond à la cible mutée que l'on vient de modéliser. On passe ensuite à l'étape 130.

Dans cette étape 130, on extrait les informations représentatives de la cible de référence.

On procède alors dans une étape 140 à un nouveau test destiné à déterminer si la cible reçue est répertoriée dans les multiplets. Si la cible n'est pas répertoriée dans les multiplets, on procède dans une étape 142 à la recherche dans la seconde table de correspondance cibles/ligands du ou des ligands qui sont susceptibles d'interagir avec la cible de référence extraite à l'étape 130. En effet, lorsque la cible mutée reçue correspond à la cible de référence déterminée (ou extraite), on peut raisonnablement supposer que les ligands qui sont réputés interagir avec la cible de référence constituent de bons candidats à l'interaction avec la cible mutée reçue.

Une fois les ligands déterminés, on procède dans une étape 144, au calcul d'une valeur d'affinité Vi pour chaque ligand Li. On obtient alors les valeurs V1, V2, ... , VN, lorsque N ligands ont été déterminés à l'étape 142.

Dans une étape 146, on extrait des multiplets associés à la cible de référence et à chacun des ligands, le ou les paramètres de référence associés Ri (R1, R2, ..., RN). Puis l'on passe à une étape 170 qui sera décrite plus loin.

Lorsqu'à l'étape 140, la cible mutée reçue a été trouvée dans au moins un multiplet, on passe à une étape 150 dans laquelle on effectue un test destiné à déterminer si dans ce ou ces multiplets se trouve stockée une valeur d'affinité Vi en complément du paramètre de référence Ri.

Si le multiplet désigné à l'étape 140 comporte effectivement une valeur d'affinité Vi et un paramètre de référence Ri, alors on passe à une étape 160 dans laquelle on extrait cette valeur d'affinité Vi et ce paramètre de référence Ri, puis l'on passe à l'étape 170.

En revanche, si le multiplet désigné à l'étape 140 ne comporte pas de valeur d'affinité, alors on procède dans une étape 152 au calcul de cette valeur d'affinité Vi puis l'on extrait du multiplet le paramètre de référence Ri qu'il contient et l'on passe à l'étape 170. Bien entendu, si plusieurs multiplets ont été désignés à l'étape 140, on détermine pour chaque ligand Li désigné, sa valeur d'affinité Vi avec la cible mutée reçue et son paramètre de référence Ri.

On retourne maintenant au test effectué à l'étape 110. Si le résultat de ce test indique qu'une cible et un ou plusieurs ligands ont été sélectionnés à l'étape 100, alors on effectue dans une étape 112 un test destiné à déterminer s'il existe un ou plusieurs multiplets qui correspondent à la cible sélectionnée et au(x) ligand(s) sélectionné(s). Si ce test indique qu'il existe effectivement des multiplets correspondant à la cible et au ligand sélectionnés, alors on passe à l'étape 150 décrite précédemment. En revanche, s'il n'existe pas de multiplet correspondant à la cible et au ligand sélectionnés ou bien s'il n'existe pas autant de multiplets qu'il y a de ligands sélectionnés, on procède dans une étape 114 à la recherche d'une cible de référence associée à la cible sélectionnée (et/ou reçue) à l'intérieur de la première table de correspondance. Puis, on passe à l'étape 152 décrite précédemment de manière à déterminer pour chaque couple cible/ligand sélectionné une valeur d'affinité Vi et un paramètre de référence Ri.

On revient maintenant à l'étape 170 dans laquelle on positionne un compteur à la valeur i=1 (pour indiquer que l'on effectue la première comparaison d'une série de N, lorsque N ligands ont été sélectionnés) et l'on extrait l'énergie d'association cible de référence/ligand ($E_{A0}$) afin de lui soustraire l'énergie d'association cible mutée/ligand ($E_{A1}$), ce qui donne $\Delta E$ (= $E_{A0} - E_{A1}$). On détermine ensuite le paramètre d'affinité Pi à partir de la relation qui le lie à $\Delta E$.

Puis, dans une étape 180 on compare le paramètre de référence Ri au paramètre d'affinité Pi pour déterminer la donnée d'affinité Di. Comme indiqué précédemment, plusieurs modes de comparaison peuvent être envisagés dès lors qu'ils font intervenir un paramètre de référence choisi et la valeur d'affinité calculée par le module de calcul et représentative de l'énergie d'association cible/ligand.

Puis, dans une étape 190 on incrémente la valeur du compteur d'une unité (i = i +1).

On effectue alors dans une étape 200, un test destiné à déterminer si d'autres comparaisons doivent être effectuées. Si tel est le cas, on retourne à l'étape 170. Dans le cas contraire, on délivre dans une étape 210 la donnée d'affinité Di, par exemple sur le moniteur du terminal d'interrogation de l'utilisateur et/ou sur son imprimante, soit directement lorsque le dispositif selon l'invention est intégré dans le terminal de cet utilisateur, soit via le réseau lorsque le dispositif selon l'invention est intégré dans un terminal de service.

Bien entendu, le procédé qui vient d'être décrit en référence à la figure 3 n'est qu'un exemple. Il pourra comporter de nombreuses variantes et/ou options destinées à raffiner certaines étapes ou bien à en accélérer les traitements.

L'invention a été présentée ci-avant en référence à une application particulière dans le domaine médical, à savoir la détermination d'un inhibiteur de la protéase du VIH-1. Mais, l'invention s'applique plus généralement à toutes les entités cible/ligand dans lesquelles le ligand est capable de s'associer à la cible, et par conséquent pour lesquelles peuvent être calculées des énergies d'association cible/ligand. C'est notamment le cas de certains inhibiteurs de la transcriptase inverse du VIH-1 qui sont capables de l'empêcher d'agir dans la phase d'association de l'ARN et de l'ADN et dans la phase de duplication. On citera par exemple les molécules de la famille « TIBO » qui comportent des complexes d'association, et notamment la nevirapine (ou BI-RG-587 ou viramune), l'efavirenz (ou DMP-266 ou L-743,726 ou sustiva) et la delavirdine (ou BHAP der ou U90152S ou rescriptor) qui viennent se coller sur la transcriptase inverse pour l'empêcher d'agir.

Mais l'invention concerne de nombreux autres domaines, et notamment tous ceux impliquant des molécules, des cellules d'un organisme humain, animal ou végétal infectées, des agents pathogènes, d'autres agents microbiens tels que les parasites, les champignons, les autres virus, les bactéries, les agents transmissibles non conventionnels (ATNC), comme par exemple le prion, et tous les ligands dirigés contre ces cibles, ainsi que tout agent étranger à un organisme et devant faire l'objet de traitement avec des molécules.

Par exemple, les molécules pourront être des protéines, des acides nucléiques et des complexes comprenant des protéines, des acides nucléiques, des sucres, des combinaisons glucido-lipido-protéiques et d'autres composés construits sur une base structurale comportant des glucides, des lipides, des amino-acides et/ou des acides nucléiques, le tout pouvant être considéré seul ou en combinaison, ainsi que des PNA (acronyme anglais de Peptide Nucleic Acid, pour lesquels des définitions peuvent être obtenues, par exemple, sur le site web dont l'adresse est http://www.bostonprobes.com).

Par ailleurs, il a été question d'un exemple dans lequel les cibles sont des mutations d'une cible de référence. Mais, il pourrait en être autrement, notamment lorsque les domaines impliqués sont la chimie combinatoire et le criblage à haut débit (High Throughput Screening), domaines dans lesquels on identifie des principes actifs de médicaments.

D'autre part, l'invention peut être utilisée pour découvrir des ligands, par exemple des médicaments. En effet, connaissant la résistance R1 et/ou l'énergie d'association $\Delta E1$ d'une cible mutée vis à vis d'un premier ligand, on peut déterminer un second ligand, résultant d'une modification du premier ligand, et présentant une résistance R2 et/ou une énergie d'association $\Delta E2$ avec cette cible mutée inférieure(s) à R1 et/ou $\Delta E1$. L'invention peut être également utilisée pour modifier une cible à l'aide d'un ligand choisi. En effet, il est possible d'utiliser un premier ligand pour induire des modifications (par exemple des mutations) dans une cible, de manière à pouvoir utiliser cette cible, modifiée par le premier ligand, avec un second ligand qui présente une résistance R2 et/ou une énergie d'association $\Delta E2$ favorable (s).

L'invention ne se limite pas aux modes de réalisation de dispositif et de procédé décrits ci-avant, seulement à titre d'exemple, mais elle englobe toutes les variantes que pourra envisager l'homme de l'art dans le cadre des revendications ci-après.

**Revendications**

1. Dispositif de détermination d'une donnée d'affinité entre un ligand et une cible, **caractérisé en ce qu'**il comprend :

   * des moyens de calcul propres à recevoir des informations représentatives d'une cible et d'un ligand susceptible d'interagir avec ladite cible en un site récepteur, et à déterminer une valeur d'affinité représentative de l'énergie d'association entre la cible et le ligand à partir desdites informations reçues, et
   * des moyens de comparaison agencés pour délivrer une donnée représentative d'un niveau d'affinité entre la cible et le ligand à partir de la valeur d'affinité cible/ligand et d'un paramètre de référence choisi.

2. Dispositif selon la revendication 1, **caractérisé en ce que** les moyens de calcul sont agencés pour déterminer l'énergie d'association entre une cible et un ligand à partir du calcul de l'énergie libre de cette cible et de l'énergie libre de l'association de cette cible avec le ligand.

3. Dispositif selon l'une des revendications 1 et 2, **caractérisé en ce qu'**il comprend une mémoire propre à stocker une pluralité de multiplets comportant au moins une information représentative d'une cible, une information représentative d'un ligand et un paramètre de référence pour l'association cible/ligand, et **en ce que** lesdits moyens de comparaison sont agencés, à réception d'une valeur d'affinité cible/ligand, pour extraire de la mémoire le paramètre de référence correspondant à l'association cible/ligand de manière à effectuer la comparaison.

4. Dispositif selon la revendication 3, **caractérisé en ce que** certains au moins des multiplets comprennent une valeur d'affinité choisie dans un groupe comprenant une énergie d'association cible/ligand, une différence d'énergie d'association, une vitesse d'association cible/ligand et une durée d'association cible/ligand.

5. Dispositif selon l'une des revendications 3 et 4, **caractérisé en ce que** la mémoire est propre à stocker dans une première table des premières cibles, dites de référence, en correspondance de listes de secondes cibles, résultant de modifications de premières cibles de référence.

**6.** Dispositif selon la revendication 5, **caractérisé en ce que** les moyens de calcul sont agencés pour recevoir des informations représentatives de secondes cibles résultant d'une modification d'une première cible de référence, dites cibles modifiées, et d'au moins un ligand susceptible d'interagir avec ladite seconde cible, extraire de la mémoire la première cible associée à cette seconde cible, puis déterminer des première et seconde valeurs d'affinité représentatives respectivement de l'énergie d'association entre la première cible et le ligand et de l'énergie d'association entre la seconde cible et le ligand, et enfin délivrer le résultat d'une soustraction entre lesdites première et seconde valeurs d'affinité, et

en ce que les moyens de comparaison sont agencés pour délivrer une donnée représentative d'un niveau d'affinité entre la seconde cible et le ligand à partir du résultat de la soustraction et d'un paramètre de référence choisi.

**7.** Dispositif selon la revendication 6, **caractérisé en ce que** le paramètre de référence est celui de l'association première cible/ligand.

**8.** Dispositif selon l'une des revendications 6 et 7, **caractérisé en ce que** les moyens de calcul sont agencés pour appliquer au résultat de la soustraction une fonction d'affinité de manière à délivrer un paramètre d'affinité, et **en ce que** les moyens de comparaison sont agencés pour comparer ledit paramètre d'affinité au paramètre de référence choisi.

**9.** Dispositif selon la revendication 8, **caractérisé en ce que** la comparaison est une opération choisie dans un groupe comprenant une soustraction et un rapport entre le paramètre d'affinité et le paramètre de référence.

**10.** Dispositif selon la revendication 9, **caractérisé en ce que** la donnée représentative du niveau d'affinité est proportionnelle au résultat de ladite opération.

**11.** Dispositif selon l'une des revendications 9 et 10, **caractérisé en ce que** la donnée représentative du niveau d'affinité cible/ligand est une information choisie dans un groupe comprenant les avertissements « mauvaise association » ou « forte résistance », correspondant à un résultat du rapport de comparaison strictement supérieur à 1 (un), « très bonne association » ou « faible résistance », correspondant à un résultat du rapport de comparaison strictement inférieur à 1 (un), et « association possible» ou « résistance possible », correspondant à un résultat du rapport de comparaison de l'ordre de 1 (un).

**12.** Dispositif selon l'une des revendications 1 à 11, **caractérisé en ce qu'**il comprend en outre des moyens de sélection propres à délivrer aux moyens de calcul les informations représentatives des cibles et des ligands.

**13.** Dispositif selon l'une des revendications 1 à 12, **caractérisé en ce qu'**il comprend un terminal informatique de service muni d'une interface de communication couplée aux moyens de sélection et/ou aux moyens de calcul, ainsi qu'à un réseau de communication, choisi parmi les réseaux publics et les réseaux privés, et propre à recevoir d'un terminal d'interrogation des données représentatives de la cible sélectionnée et/ou des données représentatives du ligand sélectionné.

**14.** Dispositif selon l'une des revendications 1 à 12, **caractérisé en ce qu'**il est susceptible d'être implanté dans un terminal informatique de service muni d'une interface de communication destinée à alimenter en informations les moyens de sélection et/ou les moyens de calcul, ainsi qu'à un réseau de communication, choisi parmi les réseaux publics et les réseaux privés, et propre à recevoir d'un terminal d'interrogation des données représentatives de la cible sélectionnée et/ou des données représentatives du ligand sélectionné.

**15.** Dispositif selon l'une des revendications 13 et 14, **caractérisé en ce que** le terminal de service est agencé pour transmettre au terminal d'interrogation, via ledit réseau de communication, la donnée représentative du niveau d'affinité cible/ligand.

**16.** Dispositif selon l'une des revendications 13 à 15, **caractérisé en ce que** le terminal de service définit un site accessible par le réseau public Internet.

**17.** Dispositif selon l'une des revendications 1 à 12, **caractérisé en ce qu'**il est susceptible d'être implanté dans un terminal informatique individuel.

**18.** Dispositif selon l'une des revendications 12 à 17, **caractérisé en ce que** les données représentatives de la cible sont fournies sous la forme d'une séquence et/ou d'un phénotype.

**19.** Dispositif selon la revendication 18, **caractérisé en ce que** la mémoire est également agencée pour stocker dans une deuxième table les cibles en correspondance de leur séquence et/ou de leur phénotype, lesdites cibles étant identiques à celles stockées dans la première table, et **en ce que** les moyens de sélection sont agencés, à réception des données de séquence et/ou de phénotype, pour déterminer dans la deuxième table les informations représentatives de la cible et destinées aux moyens de calcul.

**20.** Dispositif selon l'une des revendications 1 à 19, **caractérisé en ce que** les moyens de calcul sont agencés pour alimenter les multiplets stockés dans la mémoire en valeur d'affinité accompagnée de données représentatives des cible et ligand correspondants.

**21.** Dispositif selon la revendication 20, **caractérisé en ce que** chaque valeur d'affinité stockée dans un multiplet de la mémoire est représentative d'une énergie d'association cible/ligand.

**22.** Dispositif selon l'une des revendications 20 et 21, **caractérisé en ce que** les moyens de sélection sont agencés, en présence d'une cible et d'un ligand, pour déterminer dans les multiplets de la mémoire s'il existe une valeur d'affinité stockée en correspondance de ladite cible et dudit ligand, et, lorsque cette valeur d'affinité existe, pour l'extraire et la transmettre aux moyens de comparaison et/ou moyens de calcul.

**23.** Dispositif selon l'une des revendications 6 à 22, **caractérisé en ce que** les moyens de calcul sont agencés pour déterminer dans la mémoire, à réception d'une seconde cible, la première cible et les ligands associés à cette première cible, puis extraire pour chaque association première cible/ligand son paramètre de référence.

**24.** Dispositif selon l'une des revendications 3 à 23, **caractérisé en ce qu'**il comprend un module de calcul statistique propre à recevoir d'un utilisateur un paramètre de référence correspondant à une association cible/ligand et à modifier le paramètre de référence dans le multiplet comprenant ladite association cible/ligand, stocké dans la mémoire, lorsque ledit paramètre reçu diffère du paramètre de référence stocké.

**25.** Procédé de détermination d'une donnée d'affinité entre un ligand et une cible, **caractérisé en ce qu'**il comprend les étapes suivantes :

    a. sélectionner une cible et au moins un ligand susceptible d'interagir avec la cible en un site récepteur,
    b. déterminer une valeur d'affinité représentative de l'énergie d'association entre la cible et le ligand, et
    c. délivrer une donnée représentative d'un niveau d'affinité entre la cible et le ligand à partir de la valeur d'affinité cible/ligand et d'un paramètre de référence choisi.

**26.** Procédé selon la revendication 25, **caractérisé en ce qu'**à l'étape a) on sélectionne la cible parmi une pluralité de multiplets comportant au moins une information représentative d'une cible, une information représentative d'un ligand et un paramètre de référence pour l'association cible/ligand, et à l'étape c), à réception d'une valeur d'affinité cible/ligand, on extrait de la mémoire le paramètre de référence correspondant à l'association cible/ligand pour déterminer ladite donnée d'affinité.

**27.** Procédé selon la revendication 26, **caractérisé en ce que** l'on stocke dans la mémoire une première table établissant des correspondances entre des premières cibles, dites de référence, et des listes de secondes cibles résultant de modifications de premières cibles de référence.

**28.** Procédé selon la revendication 27, **caractérisé en ce qu'**à l'étape a) on sélectionne tout d'abord une seconde cible et un ligand susceptible d'interagir avec cette seconde cible, puis on extrait de la mémoire la première cible associée à cette seconde cible, **en ce qu'**à l'étape b) on détermine des première et seconde valeurs d'affinité représentatives des énergies d'association respectivement entre la première cible et le ligand et entre la seconde cible et ledit ligand, puis on effectue une soustraction entre lesdites première et seconde valeurs d'affinité, et **en ce qu'**à l'étape c) on délivre une donnée représentative d'un niveau d'affinité entre la seconde cible et le ligand à partir du résultat de la soustraction et d'un paramètre de référence choisi.

**29.** Procédé selon la revendication 28, **caractérisé en ce que** le paramètre de référence est celui de l'association première cible/ligand.

**30.** Procédé selon l'une des revendications 28 et 29, **caractérisé en ce qu'**à l'étape c) on applique au résultat de la soustraction une fonction d'affinité de manière à délivrer un paramètre d'affinité, puis on compare ledit paramètre d'affinité au paramètre de référence choisi pour déterminer ladite donnée d'affinité.

**31.** Procédé selon l'une des revendications 25 à 30, **caractérisé en ce qu'**à l'étape a) la cible et/ou le ligand sont transmis par un terminal d'interrogation via un réseau de communication choisi parmi les réseaux publics et les réseaux privés.

**32.** Procédé selon la revendication 31, **caractérisé en ce que** la cible est adressée sous forme d'une séquence ou d'un phénotype, et **en ce qu'**à l'étape a) on détermine la cible correspondant à la séquence ou au phénotype reçu dans une seconde table, stockée dans la mémoire, établissant une correspondance entre les cibles et leur séquence et/ou leur phénotype correspondant.

**33.** Procédé selon l'une des revendications 31 et 32, **caractérisé en ce qu'**il comprend une étape d) dans laquelle on transmet au terminal d'interrogation, via ledit réseau de communication, la donnée représentative du niveau d'affinité entre la cible et le ligand sélectionnés.

**34.** Procédé selon l'une des revendications 25 à 33, **caractérisé en ce qu'**à l'étape b) on stocke dans les multiplets chaque valeur d'affinité déterminée, en correspondance des cible et ligand associés, de manière à constituer une base de données.

**35.** Procédé selon la revendication 34, **caractérisé en ce qu'**à l'étape a) on détermine dans les multiplets s'il existe une valeur d'affinité stockée en correspondance des cible et ligand sélectionnés, de manière à passer directement à l'étape c) lorsque cette valeur d'affinité existe.

**36.** Procédé selon l'une des revendications 25 à 35 **caractérisé en ce qu'**à l'étape b), à réception d'une cible sélectionnée, on recherche dans les multiplets tous les ligands associés, puis on détermine une valeur d'affinité pour chaque association cible/ligand trouvée, et à l'étape c) on extrait le paramètre de référence de chaque association cible/ligand trouvée pour procéder aux comparaisons de chaque valeur d'affinité au paramètre de référence correspondant.

**37.** Application des dispositif et procédé selon l'une des revendications précédentes aux cibles sélectionnées dans un groupe comprenant les molécules, les cellules d'un organisme humain, animal ou végétal infectées et les agents pathogènes.

**38.** Application selon la revendication 37, **caractérisée en ce que** les agents pathogènes sont des virus choisis dans un groupe comprenant le virus d'immuno-déficience humaine (VIH-1) et le virus de l'hépatite B (VHB).

**39.** Application selon l'une des revendications 37 et 38, **caractérisée en ce que** les molécules sont choisies dans un groupe comprenant les protéines, les acides nucléiques et les complexes comprenant des protéines et des acides nucléiques et les PNA.

**40.** Application des dispositif et procédé selon l'une des revendications 1 à 36 aux sites récepteurs mutés.

**41.** Application des dispositif et procédé selon l'une des revendications 1 à 36 à l'identification de principes actifs de médicaments.

**FIG. 1**

FIG. 2

FIG. 3A

Sélection données — 100

Données cible + ligand ? — 110

Cible → Cible connue dans 1ère table — 120

NON → Modélisation cible — 122

Recherche cible de référence dans 1ère table et multiplets — 124

OUI

Extraction de cible de référence — 130

Cible connue dans Multiplets ? — 140

NON → Recherche ligands dans 2e table — 142

Calcul V1......VN — 144

Extraction S1..........SN — 146

OUI

V1 et S1 connus ? — 150

NON → Calcul de V1 + Extraction de S1 — 180

OUI

Cible + Ligand

Multiplet(s) cible +ligand connu(s) ? — 160

NON → Recherche cible de référence associée à cible reçue dans la 1ère table — 170

OUI

EP 1 209 610 A1

Extraction
V1, S1

190

Comparaison
Vi, Si

200

Donnée d'affinité
Di

210

i = i + 1

220

Autre
Comparaison
Vi, Si ?

230

OUI

NON

Transmission Di

240

**FIG. 3B**

Office européen
des brevets

**RAPPORT DE RECHERCHE EUROPEENNE**

Numéro de la demande

EP 00 40 3326

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 98 54665 A (UNIV JOHNS HOPKINS) 3 décembre 1998 (1998-12-03) * abrégé; revendications 1-17 * | 1-41 | G06F19/00 |
| Y | US 5 587 293 A (KAUVAR LAWRENCE M ET AL) 24 décembre 1996 (1996-12-24) * abrégé * * colonne 2, ligne 60 - colonne 3, ligne 9 * | 1-41 | |
| Y | WO 99 06839 A (NOVALON PHARMACEUTICAL CORP ;THORP H HOLDEN (US)) 11 février 1999 (1999-02-11) * abrégé; revendications 1-14 * | 1-41 | |
| E | WO 01 36980 A (PETT CHRISTOPHER PHINEAS ;MUCENIECE RUTA (LV); LUNDSTEDT TORBJOERN) 25 mai 2001 (2001-05-25) * abrégé; revendications 1-80 * | 1-41 | |

**DOMAINES TECHNIQUES RECHERCHES (Int.Cl.7)**

G06F

Le présent rapport a été établi pour toutes les revendications

| Lieu de la recherche | Date d'achèvement de la recherche | Examinateur |
|---|---|---|
| LA HAYE | 15 novembre 2001 | Filloy García, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03 82 (P04C02)

**ANNEXE AU RAPPORT DE RECHERCHE EUROPEENNE
RELATIF A LA DEMANDE DE BREVET EUROPEEN NO.**

EP 00 40 3326

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-11-2001

| Document brevet cité au rapport de recherche | | Date de publication | Membre(s) de la famille de brevet(s) | | Date de publication |
|---|---|---|---|---|---|
| WO 9854665 | A | 03-12-1998 | EP | 1025521 A1 | 09-08-2000 |
| | | | US | 6226603 B1 | 01-05-2001 |
| | | | WO | 9854665 A1 | 03-12-1998 |
| | | | US | 2001000807 A1 | 03-05-2001 |
| US 5587293 | A | 24-12-1996 | AU | 700497 B2 | 07-01-1999 |
| | | | AU | 1522195 A | 01-08-1995 |
| | | | CA | 2178096 A1 | 13-07-1995 |
| | | | EP | 0738390 A1 | 23-10-1996 |
| | | | JP | 9507300 T | 22-07-1997 |
| | | | WO | 9518969 A1 | 13-07-1995 |
| | | | US | 5798275 A | 25-08-1998 |
| | | | US | 5741653 A | 21-04-1998 |
| WO 9906839 | A | 11-02-1999 | AU | 8678198 A | 22-02-1999 |
| | | | EP | 1002235 A1 | 24-05-2000 |
| | | | WO | 9906839 A1 | 11-02-1999 |
| WO 0136980 | A | 25-05-2001 | AU | 1530501 A | 30-05-2001 |
| | | | WO | 0136980 A2 | 25-05-2001 |

EPO FORM P0460

Pour tout renseignement concernant cette annexe : voir Journal Officiel de l'Office européen des brevets, No.12/82